# EUROPEAN PATENT APPLICATION

(11) **EP 4 846 996 A1**
(43) Date of publication of application: **02.12.2026**
(21) Application number: 25746536.9
(22) Date of filing: 11.04.2025
(51) Int. Cl.: A61H 1/02

(54) **KNEE JOINT EXOSKELETON MOTION RECOGNITION METHOD AND KNEE JOINT ASSIST DEVICE**

(71) Applicant: Yuanye Technology (Wuxi) Co., Ltd., Wuxi, Jiangsu 214000 (CN)
(72) Inventor: DING, Ye, Wuxi, Jiangsu 214000 (CN); LENG, Peimin, Wuxi, Jiangsu 214000 (CN); LIU, Zihao, Wuxi, Jiangsu 214000 (CN); WANG, Qun, Wuxi, Jiangsu 214000 (CN); DONG, Yue, Wuxi, Jiangsu 214000 (CN); WANG, Xujun, Wuxi, Jiangsu 214000 (CN); WANG, Cunjin, Wuxi, Jiangsu 214000 (CN); WEI, Zhuoyi, Wuxi, Jiangsu 214000 (CN); LI, Xinhui, Wuxi, Jiangsu 214000 (CN)
(74) Representative: Bayramoglu et al.
(86) International application number: PCT/CN2025/088382
(87) International publication number: WO 2026/213844

(57) **Abstract**

The present disclosure relates to the field of exoskeleton assistance devices, and in particular to a knee joint exoskeleton motion recognition method and a knee joint assistance device. The knee joint assistance device includes a lower-limb exoskeleton device, where the lower-limb exoskeleton device includes: a joint drive motor including a pair of rotating assemblies and configured to apply a torque to the pair of rotating assemblies configured to rotate relatively to each other, where a center of relative rotation of the rotating assemblies corresponds to a knee joint; a thigh extension frame and a shank extension frame connected to the pair of rotating assemblies, respectively; a thigh strapping device disposed on the thigh extension frame and strapped to a thigh; and a first shank strapping device and a second shank strapping device that are disposed on the shank extension frame and strapped between a calf and the knee joint. The present disclosure has a slippage prevention effect and excellent adaptability in use, and can protect the knee during downhill motions by employing simulated damping control.

## Description

### TECHNICAL FIELD

The present disclosure relates to the field of exoskeleton assistance devices, and in particular to a knee joint exoskeleton motion recognition method and a knee joint assistance device.

### BACKGROUND TECHNOLOGY

A knee joint exoskeleton device is strapped to a user's thigh and shank, and drives the lower limb to perform assistive motions using a motor located at the knee.

Existing lower-limb knee joint exoskeletons struggle to accurately recognize the exoskeleton's motion state solely through sensors located on the lower limb. To solve this problem, additional position detection sensors must be disposed outside the lower-limb exoskeleton, resulting in cumbersome wear. Alternatively, the lower-limb exoskeleton must extend above the waist and hips, with the position detection sensors disposed above the waist and hips corresponding to the lower-limb exoskeleton, making the lower-limb knee joint exoskeleton overly bulky.

### CONTENT OF THE INVENTION

To overcome shortcomings in the prior art, the present disclosure aims to provide a device that improves the motion state recognition accuracy, facilitates wearability, and features lightweight design.

The above objective of the present disclosure is achieved by the following technical solutions:
A knee joint exoskeleton motion recognition method is based on a lower-limb exoskeleton device, where the lower-limb exoskeleton device includes: a thigh extension frame and a shank extension frame strapped to a thigh and a shank, respectively; the thigh extension frame and the shank extension frame are relatively rotatable around a knee joint; the lower-limb exoskeleton device is provided with a motion parameter sensor; and the knee joint exoskeleton motion recognition method includes a following process:
acquiring, by the motion parameter sensor, a motion parameter of the lower-limb exoskeleton device to determine a motion feature, and determining a motion state based on the motion feature.
The motion feature includes a knee joint angle, a knee joint angular velocity, a tilt angle of the shank and/or thigh, an angular velocity of the shank and/or thigh, an acceleration of the thigh and/or shank , and a hip projection deviation; and the hip projection deviation is defined as a change in a hip position at a current state relative to a preset initial posture of a lower limb.

A knee joint assistance device includes: a lower-limb exoskeleton device, wherein the lower-limb exoskeleton device includes:
a joint drive motor, where the joint drive motor includes a pair of rotating assemblies, and the joint drive motor is configured to apply a torque to the pair of rotating assemblies configured to rotate relatively to each other; and a center of relative rotation of the rotating assemblies corresponds to a knee joint; and
a thigh extension frame and a shank extension frame connected to the pair of rotating assemblies, respectively, wherein the thigh extension frame and the shank extension frame are strapped to a thigh and a shank, respectively.

As can be seen from the above technical solutions, the present disclosure has the following beneficial effects:
In the present disclosure, the motion parameter sensors (e.g., inertial measurement unit (IMU), encoder) on the lower-limb exoskeleton directly acquire the knee joint angle, angular velocity, thigh/calf tilt angle, and acceleration values. Additionally, the hip projection deviation (a geometrically calculated spatial displacement change of the lower limb) is combined to achieve comprehensive motion state recognition.

The hip projection deviation is derived through trigonometric relationships between the lower-limb length and the joint angle, replacing the function of traditional sensors at the waist/hip position and avoiding upward extension of the device to the waist/hip, significantly simplifying the structure.

All sensors are integrated on the thigh extension frame and the shank extension frame of the lower-limb exoskeleton, eliminating the need for additional waist/hip strapping components, thereby reducing wear steps and device weight.

### DESCRIPTION OF THE DRAWINGS

FIG. 1 is a schematic diagram of a knee joint assistance device in use in an embodiment of the present disclosure;
FIG. 2 is a front structural view of the knee joint assistance device (with strapping components hidden) in an embodiment of the present disclosure;
FIG. 3 is a rear structural view of the knee joint assistance device (with the strapping components hidden) in an embodiment of the present disclosure; and

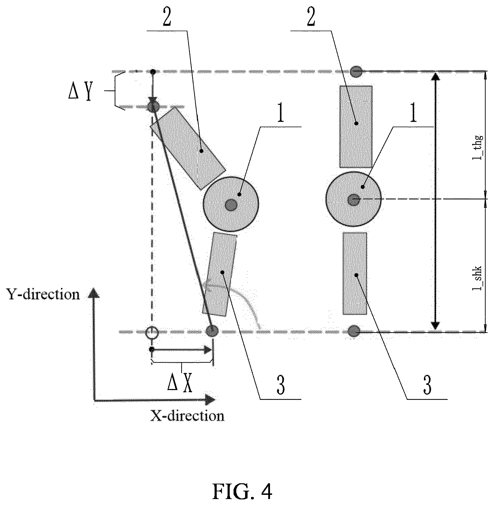

FIG. 4 is a schematic diagram illustrating a principle of a hip projection deviation in an embodiment of the present disclosure.

Reference Numerals: 1. joint drive motor; 2. thigh extension frame; 21. thigh strapping device; 211. upper connection seat; 212. first strapping component; 3. shank extension frame; 31. first shank strapping device; 311. lower connection seat; 312. second strapping component; 32. second shank strapping device; 321. knee connection seat; and 322. third strapping component.

### SPECIFIC IMPLEMENTATIONS

### Embodiment 1

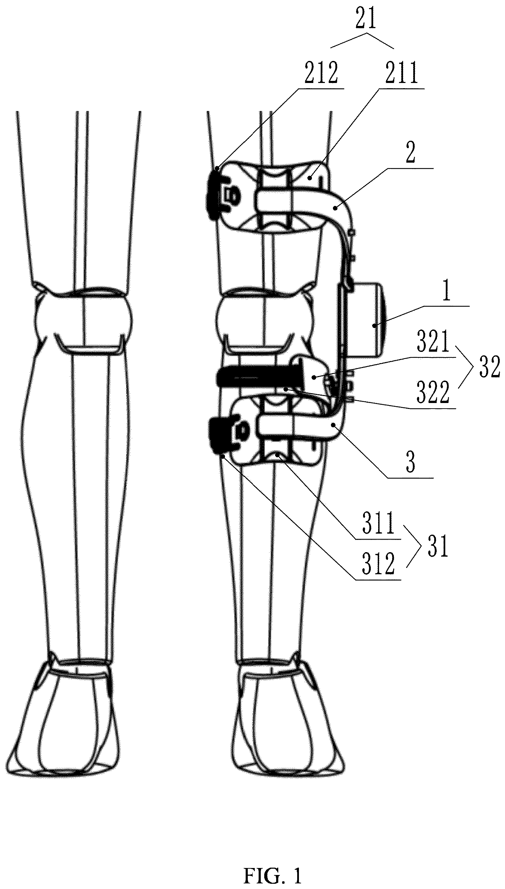

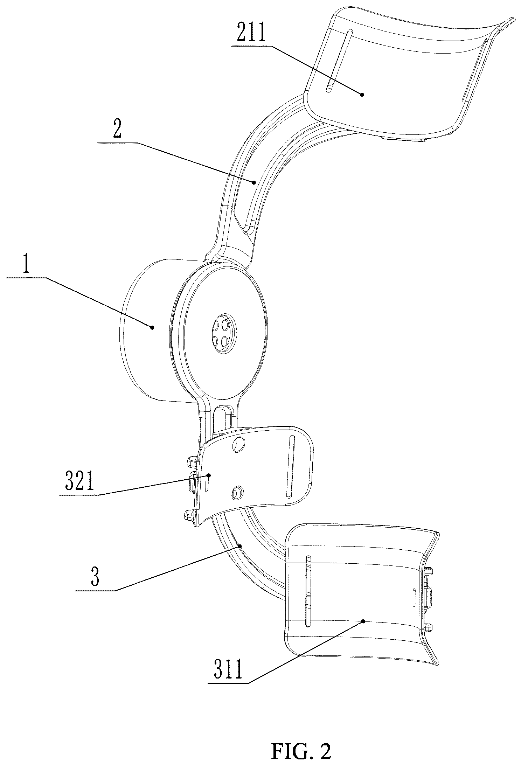

As shown in FIG. 1 to FIG. 3, this embodiment provides a knee joint assistance device, including: a lower-limb exoskeleton device.

The lower-limb exoskeleton device includes joint drive motor 1. The joint drive motor 1 includes a pair of rotating assemblies. The joint drive motor 1 is configured to apply a torque to the pair of rotating assemblies configured to rotate relatively to each other, and a center of relative rotation of the rotating assemblies corresponds to a knee joint. Thigh extension frame 2 and shank extension frame 3 are connected to the pair of rotating assemblies, respectively. Thigh strapping device 21 is disposed on the thigh extension frame 2 and is strapped to a thigh. Specifically, in this embodiment, the pair of rotating assemblies serves as a stator and rotor of the joint drive motor 1, respectively. Additionally, motion parameter sensors are disposed on the lower-limb exoskeleton device. The motion parameter sensors are configured to acquire motion parameters of the lower-limb exoskeleton device so as to determine motion features.

Existing knee joint exoskeletons tend to slip when strapped to the lower limbs and require adjustment of strapping positions for users with different leg lengths, resulting in poor adaptability. To address this problem, the knee joint assistance device in this embodiment further includes first shank strapping device 31 and second shank strapping device 32 disposed on the shank extension frame 3. The first shank strapping device 31 and the second shank strapping device 32 are strapped between a calf and the knee joint.

The working principle of the knee joint assistance device with the above structure is as follows. The joint drive motor 1 is configured to apply a torque to the rotating assemblies, transmitting a force to the thigh and the shank through the thigh extension frame 2 and the shank extension frame 3 to assist knee joint motion. In the present disclosure, the first shank strapping device 31 and the second shank strapping device 32 are arranged above the calf and below the knee joint. The thigh strapping device 21, the first shank strapping device 31, and the second shank strapping device 32 form three anchor points connecting the lower-limb exoskeleton device to the lower limb. In this embodiment, the bulging calf provides anti-slip resistance, while the short distance between the calf and the knee joint allows the two anchor points at the shank to adapt to users with different leg lengths, improving adaptability. In an embodiment, to further prevent slippage, the lower-limb exoskeleton device may be strapped to the waist or above.

Furthermore, in this embodiment, the thigh strapping device 21 includes upper connection seat 211 and first strapping component 212. The upper connection seat 211 is rotatably disposed on the thigh extension frame 2. A rotation axis of the upper connection seat 211 is horizontally oriented. The upper connection seat 211 is attached to a posterior side of the thigh. The first strapping component 212 straps the thigh to the upper connection seat 211.

Furthermore, in this embodiment, the first shank strapping device 31 includes lower connection seat 311 and second strapping component 312. The lower connection seat 311 is rotatably disposed on the shank extension frame 3. A rotation axis of the lower connection seat 311 is horizontally oriented. The lower connection seat 311 is attached to a posterior side of the shank. The second strapping component 312 straps the shank to the lower connection seat 311. The rotatable lower connection seat 311 and upper connection seat 211 are both well attached to the lower limb.

Furthermore, in this embodiment, the second shank strapping device 32 includes knee connection seat 321 and third strapping component 322. The knee connection seat 321 is disposed on the shank extension frame 3. The knee connection seat 321 is attached to a lateral side of the shank. The third strapping component 322 straps the shank to the knee connection seat 321. The knee connection seat 321 is located above the lower connection seat 311. Specifically, the strapping component is a strap connected to the connection seat via a magnetic buckle.

Furthermore, in this embodiment, the joint drive motor 1 is located at a lateral side of the knee joint. The thigh extension frame 2 and the shank extension frame 3 are each an integrally curved plate. An end of the thigh extension frame 2 and an end of the shank extension frame 3 away from the joint drive motor 1 extend to a posterior side of a leg. The thigh strapping device 21 and the first shank strapping device 31 are disposed on the end of the thigh extension frame 2 and the end of the shank extension frame 3 away from the joint drive motor 1, respectively. This design offers rational structure and lightweight advantages.

Furthermore, in this embodiment, a power module is further provided. The power module is electrically connected to the joint drive motor 1, and the power module is located at or above the waist.

Furthermore, in this embodiment, the motion parameter sensors are further provided. There are two motion parameter sensors configured to acquire motion parameters of the lower-limb exoskeleton device so as to determine at least one motion feature of knee joint angle, thigh/calf tilt angle, angular velocity, and hip projection deviation. This design minimizes the number of sensors required, reducing costs.

In an embodiment, the motion parameter sensors are a pair of inertial measurement units (IMUs) disposed on the thigh extension frame 2 and the shank extension frame 3, respectively. The knee joint angle can be calculated based on motion data such as tilt angles and angular velocities acquired by the pair of IMUs.

In another embodiment, the motion parameter sensors include an IMU disposed on the thigh extension frame 2 or the shank extension frame 3 and a knee joint angle sensor. The motion state (e.g., angular velocity, acceleration) of the extension frame without the IMU can be calculated based on motion data acquired by the IMU and knee joint angle changes acquired by the knee joint angle sensor. In an embodiment, the knee joint angle sensor is an encoder disposed on the joint drive motor 1. The encoder is configured to monitor angle changes of the stator and rotor of the joint drive motor 1, thereby deriving knee joint angle and angular velocity change signals.

### Embodiment 2

Based on the knee joint assistance device provided in Embodiment 1, the present disclosure provides a control method.

The control method includes the following steps.

The motion parameter sensors perform data processing, detection data input by the motion parameter sensors arranged on the lower-limb exoskeleton is acquired, and the detection data is processed to extract motion features.

A motion state is determined according to the motion features.

Specifically, the motion state includes a gait phase and an activity type.

Furthermore, in an embodiment, the control method further includes the following steps.

Gait phase detection is performed. A current gait phase is determined according to the motion features. The gait phase includes a stance phase and a swing phase. The stance phase corresponds to a state where the lower limb contacts the ground. The swing phase corresponds to a state where the lower limb leaves the ground.

Activity recognition is performed. The motion features during the stance phase are extracted to determine the activity type. The activity type includes at least one of walking, ascending, descending, running, squatting, backward walking, and unknown.

A control strategy is selected. The corresponding control strategy is selected according to the gait phase and the activity type to control the joint drive motor 1.

The standing stance phase (hereinafter referred to as "stance phase") is defined as a gait phase where the lower limb contacts the ground, which usually marks the beginning of a complete gait cycle. This phase occurs universally regardless of the wearer's activity type (e.g., walking, ascending, squatting, or descending). The gait always starts with foot-ground contact. Therefore, the stance phase provides a natural time window that serves as the starting point of the gait cycle for capturing and analyzing initial gait features.

Moreover, during the stance phase, the data (e.g., acceleration acquired by the thigh IMU and the knee joint angular velocity) acquired by the motion parameter sensors exhibits relatively stable trends. For example, the acceleration approaches gravitational acceleration (approximately 9.8 m/s²), and the angular velocity remains small amplitude (typically below a certain threshold, such as 0.5 rad/s). This stability provides a reliable reference signal for feature extraction, contrasting with fluctuations in subsequent dynamic phases (e.g., the swing phase).

On one hand, the extraction of motion features relies on key representations of motion patterns, and the stance phase becomes the optimal time point for recognizing activity types due to its specific biomechanical and dynamic properties. Specifically:
I. During the stance phase, the wearer's body posture (e.g., knee joint angle, shank tilt angle, hip position) directly reflects the activity type. For example, the knee joint angle decreases significantly when the wearer squats (e.g., less than 120°), while the shank tilt angle increases when the wearer ascends (e.g., exceeding 30°). These postural features are most distinct and stable when the foot contacts the ground, facilitating capture through data of the motion parameter sensors. In contrast, during the swing phase, the foot leaves the ground, and posture changes are more driven by inertia, making it hard to directly correlate with specific activity types.
II. The signals of the motion parameter sensors during the stance phase exhibit lower noise levels because the foot-ground contact reduces uncertainties caused by free motion. This stability allows algorithms to more accurately extract features, such as the steadiness of acceleration, the low amplitude of knee joint angular velocity, or the static value of the knee joint angle. In the swing phase, due to the large signal fluctuations (e.g., the acceleration may vary by 10-50% due to the swing speed), features are susceptible to environmental factors (e.g., uneven ground) or individual differences (e.g., leg-swinging habits).
III. As the starting point of a gait cycle, the stance phase provides the earliest opportunity to recognize the activity type. This early recognition is critical for subsequent gait phase detection and control. For example, after recognizing an ascending activity, the algorithm can adjust swing-phase switching conditions (e.g., increasing the knee joint angular velocity threshold) to optimize phase determination for the entire gait cycle.

Furthermore, in an embodiment, the motion features include the knee joint angle, knee joint angular velocity, tilt angle of the shank and/or thigh, angular velocity of the shank and/or thigh, and acceleration of the thigh and/or shank.

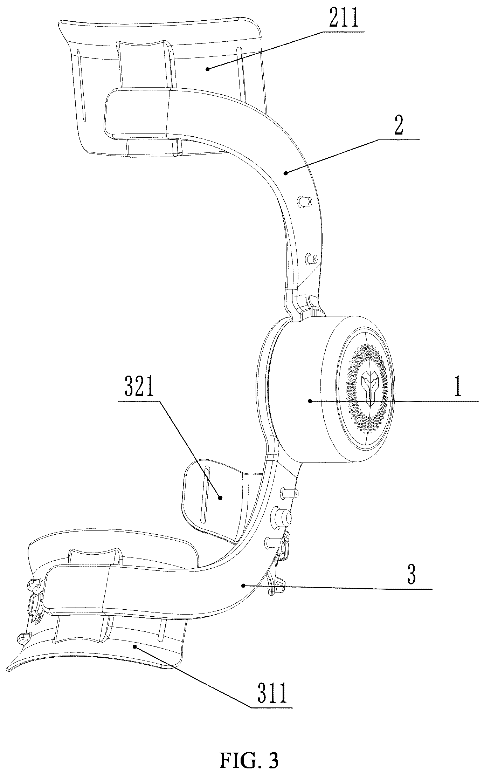

Furthermore, in an embodiment, the motion features include the hip projection deviation, which refers to a change in the hip position at the current state relative to a preset initial posture of the lower limb. Specifically, it refers to a difference in positional changes of the hip during horizontal (anteroposterior) and vertical (up-down) motion, capturing the overall trend of leg motion. Simply put, the hip motion is projected onto the ground and sagittal plane, including anteroposterior displacement (X-direction) and vertical displacement (Y-direction), and then the difference between them is calculated. This difference can characterize whether the wearer's leg tends to step forward, backward, or lift/lower more prominently. For example, during forward walking, the horizontal projection usually changes more than the vertical projection. During backward walking, the horizontal projection may decrease or even become negative, while the vertical projection may slightly increase. By measuring the hip projection deviation, different motion states can be distinguished more clearly, such as normal walking versus backward walking. Specifically, as shown in FIG. 3, when the lower limb is in the preset initial posture, the thigh and the shank are upright (e.g., the knee joint angle is 180°). The hip projection deviation includes an X-direction hip projection deviation and a Y-direction hip projection deviation.

The X-direction hip projection deviation can be expressed as the vector sum of horizontal displacement, with the forward horizontal direction as the positive X-direction, where: ΔX = - [l_shk · cos(θ_shk) + l_thg · cos(θ_thg)], where
ΔX denotes the X-direction hip projection deviation (unit: m);
l_shk and l_thg denote a length of the shank and a length of the thigh, respectively (typical values: 0.42 m each);
θ_shk and θ_thg denote an angle of the shank and an angle of the thigh relative to the vertical direction (unit: rad).
The Y-direction hip projection deviation is normalized, with the upward vertical direction as the positive Y-direction, and is expressed as ΔY, where: ΔY = l_shk · sin(θ_shk) + l_thg · sin(π - θ_thg) - L_total
ΔY denotes the Y-direction hip projection deviation (unit: m);
l_shk and l_thg denote a length of the shank and a length of the thigh, respectively (typical values: 0.42 m each);
θ_shk and θ_thg denote an angle of the shank and an angle of the thigh relative to the vertical direction (unit: rad).
sin(π - θ_thg);
L_total = l_shk + l_thg denotes the total leg length used for normalization. After normalization, it is applicable to users of different heights or leg lengths. The motion features based on the hip projection deviation also include a rate of change of the hip projection deviation.

A time derivative of the hip projection deviation is calculated, including an X-direction hip projection deviation differential and a Y-direction hip projection deviation differential of the hip joint. Then a smooth differentiator is provided to capture the rate of change of the hip projection deviation, so as to further enhance the description of motion trends.

In an embodiment, the hip projection deviation functions in gait phase detection through the following means.
1. Switching from the stance phase to the swing phase:
   During the switching from the stance phase to the swing phase, the hip projection deviation provides spatial context for the body's center of gravity and the leg position. For example, when the X-direction hip projection deviation becomes negative (e.g., less than -0.12 m) and the Y-direction hip projection deviation shows an upward shift (e.g., greater than -0.08 m), if the knee joint angular velocity exceeds 1.5 rad/s, the system determines that the leg begins to leave the ground and enters the swing phase.

The dynamic change of the hip projection deviation (first-order time derivative) further assists in determining the switching timing. For example, when the first-order derivative of the X-direction hip projection deviation > 0 and the first-order derivative of the Y-direction hip projection deviation < -0.1, it indicates hip forward motion and upward leg lifting, reinforcing swing phase recognition.

2. Switching from the swing phase to the stance phase:
During the switching from the swing phase to the stance phase, the hip projection deviation is used to detect spatial motion features of the foot-ground contact. For example, when the Y-direction hip projection deviation reaches a positive peak (e.g., greater than 0.15 m) and its first-order derivative (with respect to time) > 0.25, it indicates that the leg has completed swinging and re-contacts the ground, triggering the stance phase determination.

The extremum of the hip projection deviation is combined with a time window (e.g., within 150 ms after peak occurrence) to ensure robust transition conditions.

3. Enhancement of activity type context:
The hip projection deviation is not only used for phase transitions but also supports activity type recognition through its trends and amplitudes. For example, during ascending, the X-direction hip projection deviation is typically small (e.g., less than 0.05 m), while the Y-direction hip projection deviation shows a significant negative value (e.g., less than -0.07 m), reflecting the spatial characteristics of leg lifting. These motion features are used to dynamically adjust transition thresholds, adapting phase detection to specific activities.

Compared with other motion features, the hip projection deviation has the following unique advantages:
1. Comprehensive spatial context:
   The hip projection deviation directly reflects the two-dimensional displacement (X- and Y-directions) of the leg relative to the body's center of gravity, providing more comprehensive spatial motion information than single-joint angles or angular velocities. For example, the knee joint angle only describes local joint state, while the hip projection deviation captures the overall coordination between the hip and the leg. This global perspective is particularly important for complex activities (e.g., ascending or descending stairs), which involve a significant motion by the body center-of-gravity.
2. Sensitivity to center-of-gravity motion:
   The hip projection deviation is highly sensitive to changes in the wearer's center of gravity, effectively distinguishing between the stability of the stance phase and the dynamics of the swing phase. For example, during walking, the transition of the X-direction hip projection from negative to positive reflects the shift of the center of gravity from back to front, while the negative change in the Y-direction projection indicates the leg lift height. This dual-axis motion feature outperforms reliance on the knee joint angular velocity alone, which may lose discriminative power due to individual gait differences.
3. Robustness and environmental adaptability:
   The hip projection deviation is based on geometric calculations (trigonometric functions) and is less sensitive to sensor noise (e.g., acceleration drift in the IMU). In contrast, acceleration-based motion features are easily affected by external disturbances (e.g., uneven ground), while the hip projection deviation maintains high stability through standardized calculations using the leg length. This robustness makes it more advantageous in dynamic and variable scenarios such as mountain climbing.
4. Low computational overhead:
   The calculation of the hip projection deviation involves only basic trigonometric operations and addition/subtraction, with low time complexity and no need for complex filtering or iterative optimization. Compared with combined motion features based on acceleration and angular velocity (e.g., deviation ratios or curvature changes), the hip projection deviation provides sufficient discriminative capability while maintaining efficiency, aligning with the lightweight algorithm design goals of the present disclosure.

If the hip projection deviation is removed and the gait phase detection relies solely on other motion features, the following adverse consequences may occur.
1. Reduced accuracy of phase transitions:
   Without the spatial context of the hip projection deviation, the system may struggle to accurately determine the leg position relative to the body. For example, during descending activities, relying solely on angular velocity while ignoring the positive peak of the X-direction hip projection deviation may misinterpret brief leg adjustments as the swing phase, leading to misapplication of control strategies (e.g., erroneously applying transparent control during the stance phase).
2. Diminished capability to distinguish activity types:
   The hip projection deviation provides critical spatial motion features for activity recognition, such as the negative trend of the Y-direction hip projection deviation during ascending. Without this motion feature, the system may fail to effectively distinguish walking from ascending (whose angular velocity patterns may be similar), reducing the precision of dynamic threshold adjustments and ultimately causing mismatched assistive output.
3. Reduced robustness:
   In complex terrains (e.g., uneven ground in case of mountain climbing), angular velocity and acceleration are susceptible to noise, while the hip projection deviation maintains relative stability through geometric relationships. Without the hip projection deviation, the system's adaptability to environmental changes will significantly decrease, potentially triggering frequent unknown phase entries during anomalies (e.g., transient sensor signal distortion), compromising wearer safety and comfort.
4. Impaired transition smoothness:
   The rate of change (first-order time derivative) of the hip projection deviation provides a smooth dynamic basis for gait phase transitions. Without this motion feature, the transition conditions may rely excessively on instantaneous values (e.g., angular velocity thresholds), causing jittery or abrupt gait phase transitions that disrupt exoskeleton control continuity and user experience.

Therefore, the hip projection deviation (X- and Y-directions) significantly enhances gait phase detection accuracy and adaptability by providing the spatial context, center-of-gravity sensitivity, and robustness. Its unique advantage lies in efficiently characterizing complex motion patterns at low computational cost, enabling stable phase transitions and control output of the system across diverse activities (e.g., mountain climbing, ascending). Without the hip projection deviation, the system faces reduced accuracy, diminished adaptability, and unsmooth transitions, limiting its performance in dynamic scenarios.

In an embodiment, the hip projection deviation functions in activity type determination through the following means.

The X- and Y-direction hip projection deviations are spatial displacement metrics calculated based on the angles and lengths of the shank and the thigh, configured to characterize positional changes of the knee joint exoskeleton during motion. By incorporating the hip projection deviation and its derivative (e.g., the rate of change of the hip projection deviation over time), activity types such as descending stairs, ascending stairs, or walking can be effectively determined. These hip deviations reflect the relative motion trajectories of the hip in horizontal (X-direction) and vertical (Y-direction) dimensions, closely related to leg posture and motion dynamics.

For activity type determination, the X-direction hip projection deviation is primarily used to detect horizontal displacement characteristics. For example, in the activity of descending stairs, the X-direction hip projection deviation typically exhibits a small positive or negative offset, while its derivative can further capture dynamic motion changes to distinguish between fast and slow descending patterns. In the activity of walking, the X-direction hip projection deviation typically exhibits a periodic negative offset, reflecting a forward leg motion. In the activity of ascending, the X-direction hip projection deviation may exhibit a large positive offset, indicating a leg lift and propulsion motion.

The Y-direction hip projection deviation primarily reflects vertical displacement changes and is often used to detect height-related activities. For example, in the activity of descending stairs, the Y-direction hip projection deviation is typically negative, indicating a downward hip motion relative to the initial position. In the activity of ascending stairs, the Y-direction hip projection deviation may shift from negative to positive, reflecting a leg lift and landing process. Normalization on the Y-direction hip projection deviation (subtracting total leg length) can eliminate individual leg-length differences, enhancing universality of the determination result.

By integrating the X- and Y-direction hip projection deviations and their derivatives, the system constructs a multidimensional motion feature space. Combined with other sensor data (e.g., knee joint angle, shank/thigh angles, gyroscope data), activity type discrimination criteria are formed. This method captures spatiotemporal motion characteristics, enabling precise classification of different activity types.

Therefore, the hip projection deviation offers the following unique advantages:
1. Spatial intuitiveness. The hip projection deviation directly reflects hip displacement in the three-dimensional space, providing clearer motion trajectory information than single-angle or velocity metrics, helping distinguish activities with similar angular motion features but different spatial paths (e.g., walking vs. stair descent).
2. Dynamic adaptability. The derivative of the hip projection deviation captures acceleration and trend changes, increasing system sensitivity to dynamic activities (e.g., sudden leg lifts during fast stair ascent/descent).
3. Robustness. By synthesizing shank/thigh lengths and angles, the hip projection deviation mitigates sensor noise or individual posture variations, outperforming raw sensor data stability.
4. Multidimensional synergy. The combination of the X- and Y-direction hip projection deviations provides two-dimensional motion descriptions complementary to other motion features (e.g., knee joint angular velocity), enhancing activity classification accuracy and specificity.

If X- and Y-direction hip projection deviations are not used, activity type determination may face the following problems:
1. Decreased discriminative capability. Relying solely on single motion features such as angles or angular velocities may fail to effectively distinguish activities with similar spatial trajectories but different dynamics. For example, stair descent and walking may exhibit similar knee joint angle ranges, but their horizontal and vertical hip displacement patterns differ significantly. Therefore, removing the hip projection deviation will lead to an increase in the false recognition rate.
2. Missing dynamic information. The derivative of the hip projection deviation provides real-time change information of motion trends. Without it, the system may struggle to capture rapid motions or transition states (e.g., switching from standing to stair descent), affecting the responsiveness and stability of the control algorithm.
3. Reduced adaptability. The normalized design of the hip projection deviation accommodates users of different heights or leg lengths. Without the hip projection deviation, the system may need additional calibration steps to adapt to individual differences, increasing usage complexity.
4. Limited control precision. In knee joint exoskeleton control, the hip projection deviation provides key references for gait phase transitions and torque allocation. Without this information, the control algorithm may fail to accurately match user motion intent, causing under- or over-assistance that compromises comfort and safety.

In summary, the hip projection deviation plays an indispensable role in activity type determination, significantly enhancing knee joint exoskeleton control performance in complex terrains and dynamic activities.

Furthermore, in an embodiment, the motion features include the product of thigh acceleration and knee joint angular velocity to characterize mechanical interactions during motion. Specifically, the product of thigh acceleration and knee joint angular velocity is designed as an intermediate variable. Its core rationale is to fuse two motion parameters of different dimensions (linear acceleration and angular velocity) to extract more discriminative features, enhancing activity recognition and gait phase detection accuracy. Specific applications in some embodiments include the following aspects:
1. Characterization of mechanical interactions:
   This feature directly reflects dynamic interactions between linear thigh motion and rotational knee joint motion. For example, when the wearer lifts the leg during ascending, the thigh acceleration may exhibit a positive peak (e.g., 12 m/s²) while the knee joint angular velocity rapidly increases (e.g., 2.0 rad/s), yielding a product of 24 m•rad/s³, indicating strong mechanical coupling. During walking, the lower angular velocity (e.g., 0.8 rad/s) results in a smaller value (e.g., 9.6 m•rad/s³) despite similar acceleration. This difference provides a significant basis for differentiation in activity classification.
2. Amplification of dynamic trends:
   Through multiplication, this feature amplifies dynamic changes during the motion. For example, during rapid transitions from the swing phase to the stance phase (e.g., running landing), the thigh acceleration may drop sharply (e.g., from 15 m/s² to 9.8 m/s²) while the knee joint angular velocity approaches zero from a positive value (e.g., from 1.5 rad/s to 0.2 rad/s). The product's abrupt change (from 22.5 m•rad/s³ to 1.96 m•rad/s³) sensitively captures this trend, aiding precise determination for gait phase switching.
3. Noise suppression robustness:
   When the thigh acceleration or knee joint angular velocity is used alone, the signal may be affected by environmental noise (e.g., uneven ground-induced acceleration jitter) or sensor drift. By multiplying the two, this engineered feature can smooth instantaneous anomalies in individual variables to some extent. For example, if the acceleration exhibits an abnormal peak (e.g., 20 m/s²) due to transient interference while the knee joint angular velocity remains low (e.g., 0.3 rad/s), the product value (6 m•rad/s³) maintains within a reasonable range, avoiding false recognition.

In specific applications for activity recognition, when the motion features during the stance phase are extracted to determine the activity type, the product of the thigh acceleration and the knee joint angular velocity is used as a key discriminant indicator. In some embodiments, for example:
Walking: The product value exhibits periodic fluctuations within a typical range of 5-15 m•rad/s³, reflecting a stable gait rhythm.

Ascending: The product value significantly increases during leg lifting (e.g., 20-30 m•rad/s³) because the acceleration and the angular velocity simultaneously reach peak values.

Squatting: The product value remains small and lasts longer (e.g., 2-5 m•rad/s³, lasting about 500 ms), reflecting low-speed and high-load characteristics.

Descending: The product value remains moderate and changes smoothly (e.g., 10-20 m•rad/s³) because the angular velocity is lower while the acceleration tends to stabilize.

By setting threshold rules combined with time window analysis (e.g., average value within 300 ms), the system efficiently distinguishes different activity types.

Role in the control strategy
The product of the thigh acceleration and the knee joint angular velocity also directly influences the selection of the actuator control strategy. For example:
When exceeding a certain threshold (e.g., 25 m•rad/s³), it indicates the wearer is in a high-dynamic activity (such as running or ascending stairs), and the system can select hybrid control, combining elasticity and damping effects to provide strong assistance.

When low and stable (e.g., 3-8 m•rad/s³), it indicates the wearer is in a quasi-static state (such as squatting or standing), and the system can employ elastic control to simulate a spring effect.

Compared to the thigh acceleration and the knee joint angular velocity used alone, the product of the thigh acceleration and the knee joint angular velocity possesses the following advantages:
1. Information fusion. The product form integrates linear and rotational motion information, providing a richer mechanical description than a single variable.
2. Dynamic sensitivity. The multiplication operation amplifies the synergistic variation of both variables, making the feature more responsive to the motion intent.
3. Computational efficiency. Only one multiplication operation is required to generate the feature, complying with the lightweight algorithm design goal and suiting real-time processing in embedded systems.
4. Strong adaptability. The range and trend of the product value adapt to different wearers' motion habits (such as stride or speed differences) without requiring additional calibration.

If the product of the thigh acceleration and the knee joint angular velocity is removed and reliance is placed solely on raw variables, the following problems may occur:
Discriminative capability declines. Single variables struggle to simultaneously capture coupling effects between linear and rotational motions, potentially confusing dynamically similar activities (e.g., walking versus slow stair ascent).

Insufficient dynamic detection. The system may miss key timing of rapid transitions (e.g., from the swing phase to the stance phase) due to absence of the amplification effect on variation trends provided by the product feature.

Imprecise control. The control strategy may fail to accurately match motion demands due to lack of comprehensive mechanical indicators, resulting in insufficient or excessive assistance.

Furthermore, in an embodiment, the motion features include a normalized thigh acceleration-knee joint angular velocity ratio. Feature robustness is enhanced by applying an upper-limit threshold to constrain outlier effects.

The core of the normalized thigh acceleration-knee joint angular velocity ratio lies in the polarity of the knee joint angular velocity, indicating whether the knee joint is flexing (e.g., positive angular velocity during leg lifting) or extending (e.g., negative angular velocity during leg lowering). The system can better understand the motion intent of the wearer's leg through this directional distinction. For example, rapid knee joint flexion typically occurs during the swing phase, while slow knee joint extension or stabilization may occur during the stance phase.

On this basis, the relationship between the thigh acceleration and the knee joint angular velocity is further incorporated. Specifically, a ratio is generated by comparing the linear acceleration of the thigh against rotational speed of the knee joint. This ratio undergoes normalization rather than simple division, ensuring values remain within a controlled range while preventing inconsistencies caused by amplitude differences in raw data (e.g., acceleration typically measured in m/s² versus angular velocity in rad/s). Relative strength between both parameters is more intuitively reflected by the normalized ratio. For instance, both the thigh acceleration and the knee joint angular velocity may be high during rapid leg lifting, while both may be low during slow squatting. Additionally, an upper-limit threshold is applied to constrain extreme values caused by sensor noise, external disturbances, or abnormal wearer motions. For example, when the wear experiences a sudden external impact, the thigh acceleration may exhibit transient abnormal peaks while the knee joint angular velocity remains normal. The ratio is confined within a reasonable range by the upper-limit threshold to prevent false recognition by the system.

This feature demonstrates exceptional performance in multiple application scenarios for lower-limb exoskeletons, particularly in activity type recognition and gait phase detection. For example:
Gait analysis during walking. During normal walking, the knee joint angular velocity is positive in the swing phase (knee joint flexion), while approaching zero or negative in the stance phase (knee joint extension). The synergistic effect between the rapid increase in the thigh acceleration and the angular velocity during the swing phase is highlighted by the normalized ratio, assisting the system in precisely distinguishing the swing and stance phases.

Dynamic capture during stair ascent. During stair ascent, the knee joint angular velocity exhibits distinct positive values during leg lifting, while the thigh acceleration significantly increases due to leg lifting. This high-dynamic state is reflected by the normalized ratio, and abnormal fluctuations caused by the uneven ground or excessive wearer exertion are avoided by the upper-limit threshold.

Squatting or standing stability determination. During squatting, the knee joint angular velocity is typically negative (slow knee joint extension), and the thigh acceleration is low and stable. The normalized ratio remains a small value with smooth variations, indicating a low-speed motion state, and the system can be adjusted to a low-assistance mode accordingly.

Filtering of abnormal motions. When a sudden thigh acceleration surge occurs due to falls or external impacts while the knee joint angular velocity remains unchanged, abnormally high normalized ratio values may appear. However, the ratio is confined within a reasonable range through the intervention of the upper-limit threshold, preventing the system from falsely recognizing the motion as a high-dynamic activity.

Reasons for its enhanced robustness include the following aspects.

Polarity of the knee joint angular velocity. Different motion phases are naturally distinguished by the feature through directional focus on the angular velocity, avoiding ambiguity inherent in single values. For example, flexion and extension intent can be clearly differentiated by the polarity difference even with identical absolute angular velocity values.

Normalization. Direct influence from sensor data dimensions or individual wearer differences (e.g., weight, stride length) is eliminated for the ratio by normalization. For instance, a heavier wearer may generate a greater acceleration, but the normalized ratio aligns with trends observed in lighter wearers, enhancing feature universality.

Protective role of upper-limit threshold. The upper-limit threshold acts as a safety valve, preventing outlier interference with system determination. For example, if an instantaneous acceleration surge occurs during running due to ground protrusions, the threshold mechanism prevents ratio runaway, ensuring the system continues operating in normal running mode.

Additionally, the normalized thigh acceleration-knee joint angular velocity ratio supports control strategy optimization for the lower-limb exoskeleton. In some embodiments, for example:
When the ratio is high and the knee joint angular velocity is positive, it indicates the wearer is in a rapid leg-lifting state (e.g., running or ascending stairs), and the system can increase the assistive output of the actuator to provide greater thrust.

When the ratio is low and the angular velocity is negative, it indicates the wearer may be slowly lowering the leg (e.g., squatting or descending stairs), and the system can switch to a damping mode to enhance stability and reduce impact.

When the ratio triggers the upper-limit threshold due to outliers, the system temporarily maintains the current control strategy to avoid frequent mode switching caused by transient interference, thereby improving wear experience coherence.

Furthermore, in an embodiment, the motion features include accumulation of a positive knee joint angular velocity to quantify a sustained knee joint motion trend, where the accumulated value is reset when the knee joint angular velocity is negative.

Furthermore, in an embodiment, the motion features include maximum/minimum values of angles and angular velocities for the knee joint, the thigh, and the shank, characterizing a motion peak trend.

Furthermore, in an embodiment, the control strategy includes at least one of elastic control, damping control, and hybrid control.

The elastic control involves controlling the torque applied by the joint drive motor 1 to swing arm 11 according to the knee joint angle so as to simulate a spring.

The damping control involves controlling the torque applied by the joint drive motor 1 to the swing arm 11 according to the knee joint angular velocity so as to simulate a damper.

The hybrid control combines the elastic and damping control to adapt to current activity types.

The control strategy corresponding to the stance phase includes at least one of elastic control, damping control, and hybrid control.

Control parameters are adjusted in real time according to the current gait phase and/or activity type and/or motion features during execution of the control strategy, such as adjusting elasticity of the simulated spring or damping force of the simulated damper.

Regarding the control strategy for the standing stance phase, it should be noted that:
During the standing stance phase (hereinafter "stance phase"), the system applies a specific control strategy upon detecting motion states during foot-ground contact to optimize wearer stability, comfort, and efficiency. These strategies include elastic control, damping control, and hybrid control, each designed for different motion demands and scenarios.

For elastic control, the system applies the torque according to the joint angle to simulate behavior of a virtual spring. For example, when the wearer is in a half-squat posture (the knee joint angle is approximately 90-120°, typically during transitions from standing to squatting), if the system detects the angle change, it indicates that the leg is under a static load or a quasi-static transition. At this time, the system applies an assistive torque (e.g., 5-15 Nm, depending on the wearer weight and joint stiffness requirements) proportional to the angular deviation to reduce the leg muscle burden and simulate the natural rebound effect of the spring. The benefit of this control lies in enhancing the wearer strength through the assistance provided and reducing energy consumption during posture recovery (e.g., standing from a squat) while avoiding discomfort caused by excessive joint flexion. The joint angle is detected by the IMU, which combines accelerometer and gyroscope data to calculate relative leg angles in real time, ensuring detection accuracy and response speed.

For damping control, the system applies the torque according to the knee joint angular velocity to simulate the buffering effect of a damper. For example, when the wearer walks on an uneven ground or squats rapidly, the knee joint angular velocity may reach 0.5-1.5 rad/s, indicating the leg is undergoing a dynamic adjustment or an external disturbance (e.g., ground impact). In this scenario, the system applies a reverse torque (e.g., 3-10 Nm, depending on the knee joint angular velocity) proportional to the knee joint angular velocity to slow the joint motion speed, avoiding instability or muscle fatigue caused by excessive flexion. The advantage of this control lies in its capability to smooth motion trajectories and suppress unnecessary oscillations (e.g., jitter during walking), thereby enhancing wearer safety and comfort.

Hybrid control combines elastic and damping effects, and applies dynamic adjustments according to activity types. For example, in a scenario of carrying a heavy load while squatting, the wearer may first enter a half-squat (triggering elastic control to provide 10 Nm of spring torque assistance), followed by a rapid joint adjustment due to a load sway (the knee joint angular velocity reaches 1.0 rad/s, triggering damping control to apply 5 Nm of buffering torque). This combined strategy ensures that the system adaptively matches wearer demands in complex activities by simultaneously providing assistance and stability. Its advantage lies in integrating the strength enhancement of elastic control with the motion smoothness of damping control, adapted to diverse stance phase scenarios (e.g., ascending, standing from a squat, or load-carrying walking), thereby improving overall motion efficiency and wear experience.

The primary cause to implement these control strategies during the stance phase lies in that this gait phase is a critical timing for activity type recognition and load support. Wearer actions (e.g., walking, squatting, or standing adjustments) typically commence during the stance phase, where the knee joint angle and angular velocity provide rich motion information, enabling the system to accurately determine the activity intent and optimize the torque output.

Additionally, the stability and assistance requirements during the stance phase directly influence smoothness of the subsequent swing phase. Through the synergistic action of elastic, damping, and hybrid control, the system improves current gait performance while establishing foundations for phase transitions.

Furthermore, in an embodiment, the control strategy includes at least one of transparent control and resistance-reduction control.

The control strategy corresponding to the swing phase includes transparent control and/or resistance-reduction control.

Regarding the control strategy corresponding to the swing phase, it should be noted that:
During the swing phase, the foot leaves the ground, the leg enters a free-motion state, and the system aims to minimize intervention or provide mild enhancement to support natural motion and improve efficiency. The control strategy includes transparent control and resistance-reduction control, targeting distinct dynamic demands.

The transparent control sets the torque output to zero, permitting natural leg motion by the wearer. For example, during normal walking, when the leg enters the swing phase (e.g., from heel-off to toe-contact), the knee joint swings freely at an angular velocity of approximately 0.8-1.2 rad/s. At this time, the system does not apply any active torque. Instead, the low-friction design of the device (e.g., achieved via an efficient motor and transmission system) ensures no additional resistance is perceived by the wearer. The advantage of this control lies in maximally preserving the wearer's natural gait, avoiding discomfort or energy waste caused by unnecessary intervention, while maintaining high device transparency for daily activities (e.g., level walking or jogging).

The resistance-reduction control applies a small assistive torque to counteract friction or gravity effects. For example, during long-distance walking or uphill climbing, the knee joint in the swing phase may increase the burden on the wearer due to device weight (typically 2-5 kg) or terrain resistance. At this time, the system detects the reduction in the knee joint angular velocity (e.g., below 0.5 rad/s, indicating hindered swing), and applies a mild assistive torque (e.g., 1-3 Nm, aligned with swing direction) to compensate for resistance induced by device friction and gravity. The benefit of this control lies in reducing the wearer's muscle fatigue without altering natural motion trajectories, significantly enhancing efficiency during prolonged activities or high-intensity tasks (e.g., mountain climbing).

The swing phase selects these control strategies because the core requirements of this gait phase are freedom and efficiency. Transparent control satisfies the natural motion requirement through zero-torque output, while resistance-reduction control compensates for inherent physical limitations (e.g. mass or friction) of the device through the fine-tuned torque to optimize the wearer's experience. Additionally, the swing phase control directly impacts gait cycle coherence, and lightweight intervention strategies ensure seamless connection with the stance phase while avoiding gait disorders caused by excessive control.

Furthermore, in an embodiment, the gait phases additionally include an unknown phase, corresponding to at least one of: a transitional state with the motion features not clearly matching the stance or swing phase, a state with the algorithm unable to determine the current gait, and a state of algorithm initialization. The control strategy for the unknown phase includes transparent control and/or resistance-reduction control to ensure safety and comfort.

Regarding the control strategy for the unknown phase, it should be noted that:
When the system cannot definitively determine the current gait phase (e.g., during the unknown phase), the transparent control or resistance-reduction control is prioritized to ensure safety and comfort. This strategy applies to scenarios with ambiguous gait transitions or uncertain signals from the motion parameter sensors.

During the unknown phase, the transparent control is the system default, maintaining a zero torque output. For example, when the wearer suddenly starts walking from standing, but the initial data of the motion parameter sensors (e.g., IMU signals) cannot accurately distinguish stance from swing due to noise or brief occlusion, the system avoids applying any active torque. The motion support relies solely on passive characteristics of the device. This approach avoids risks from false recognition through zero intervention (e.g., tripping caused by accidental damping torque applied during the swing phase), thereby ensuring wearer safety and motion smoothness.

If a slight motion trend is detected (e.g., the knee joint angular velocity is 0.2-0.5 rad/s, indicating a potential swing initiation or posture micro-adjustment), the system can switch to resistance-reduction control and apply a minimal torque (e.g., 0.5-2 Nm) to support a potential motion intent. For example, during a slow stance adjustment or step preparation by the wearer, this mild assistance counteracts device friction, ensuring smooth motion initiation. This strategy provides moderate support under uncertainty while maintaining minimal intervention, avoiding disruption to the wearer.

These control strategies are adopted during the unknown phase because of safety and robustness. When the gait phase is unclear, any aggressive control (e.g., high-torque output) may cause discomfort or even danger. Therefore, prioritizing transparent control can effectively mitigate risks. The resistance-reduction control serves as a conservative supplement, ensuring system responsiveness under potential motion intent. This design not only elevates fault tolerance of the system but also maintains reliability and wearing comfort in complex or non-standard scenarios (e.g., irregular gait or sudden actions).

Furthermore, in an embodiment, the gait transition conditions and/or control parameters corresponding to the control strategies are adjusted based on the activity types and/or motion features, thereby adapting the exoskeleton to evolving scenarios.

The motion features extracted during the stance phase are utilized not only for classifying the current activity but also directly influencing subsequent gait-switching logic. Specifically:
I. The stance phase is transitioned to the swing phase. Based on the activity type recognized during the stance phase, the switching conditions are dynamically adjusted by the algorithm. For example, if "squatting" is recognized, a longer acceleration stabilization time (e.g., 500 ms) is required to confirm stance phase completion, avoiding false recognition of swing initiation due to brief standing from a squat. If "ascending" is recognized, the stabilization time may be shortened (e.g., 150 ms) with an increased knee joint angular velocity threshold (e.g., 1.5 rad/s) to accommodate rapid leg lifting. This adjustment ensures accuracy of gait phase determination and relevance of gait switching.
II. Seamless control is implemented. By extracting the motion features during the stance phase, the system can establish a motion context early in the gait cycle, thereby providing continuous instructions for control of the exoskeleton or walking aid device. For example, features recognized during a "descending" activity may trigger gentler actuator responses, avoiding abrupt changes.

It should be noted that phase transition refers to the process by which the exoskeleton system switches from one gait phase (e.g., stance phase) to another gait phase (e.g., swing phase or unknown phase) according to the real-time motion state of the wearer. This transition is achieved by detecting specific gait features (e.g., joint angle, knee joint angular velocity) and comparing them with predefined thresholds or conditions. The core logic of gait phase transition relies on fusion of data from multiple motion parameter sensors and dynamic threshold determination, ensuring accurate recognition of wearer motion intent and timely adjustment of control strategies by the system.

During the stance phase, the system continuously monitors the filtered knee joint angular velocity value. The transition to the swing phase is triggered when this parameter exceeds a preset dynamic threshold (typically 0.8 rad/s) accompanied by hip forward projection offset reaching 0.25 m. A triple-verification mechanism is adopted for this process. First, persistence of motion trends is captured by a thigh IMU. Second, instantaneous noise interference is suppressed by a joint angle extremum locking technology. Finally, confidence assessment is conducted through a phase prediction model combining historical gait data, ensuring reliability of transition determination.

The regulation of the transition logic based on the activity type is manifested in two aspects: dynamic threshold compensation and control domain remapping. Taking an ascending scenario as an example, the system increases the knee joint angular velocity determination threshold to 1.5 rad/s to compensate for the response delay caused by an increased joint load. Meanwhile, a kinematic compensation algorithm for the hip forward projection offset is introduced, dynamically correcting forward body center-of-gravity shift during stair climbing.

Furthermore, in an embodiment, the motion features additionally include frequency-domain feature of knee joint motions. In complex terrain scenarios, the system characterizes ground roughness by frequency-domain features of knee joint motion (e.g., tremor components at 2-4 Hz), dynamically coupling the elastic coefficient of the simulated spring in elastic control and the damping coefficient of the simulated damper in damping control. For example, in case of excessive terrain complexity (e.g., ground roughness surpasses a critical value), the damping coefficient is proportionally increased to its maximum value, and the elastic coefficient is reduced to 60% of its baseline value, effectively suppressing joint oscillation induced by irregular impacts.

For low-speed high-load activities like standing from a squat, the system constructs a nonlinear elastic control strategy. When the knee joint angle enters a 60-100° working range, an assistive torque exhibiting an exponential relationship to the angular rate of change is applied. Peak assistance (typically 12 Nm) is generated when the angle reaches 90°. During this process, the Y-direction hip projection deviation serves as a stance phase maintenance condition. When it is sustained beyond 0.1 m for 200 ms, the system locks current control parameters, preventing false switching caused by accidental center-of-gravity fluctuations.

The abnormal condition handling mechanism adopts a simple safety strategy. Upon entering the unknown phase, the system automatically switches to the transparent control mode.

In some embodiments, the methods for gait phase detection and gait phase switching condition control are specifically as follows.

Before the switching conditions are adjusted according to the detected activity type, the gait phase switching conditions are based on predefined rules and feature trends of sensor data, specifically as follows.

The switching condition from the swing phase to the stance phase is as follows. When the sensors detect a foot-ground contact, for example when the thigh acceleration stabilizes (e.g., approaches a typical gravity acceleration range, such as about 9.8 m/s²), and when the amplitude of the knee joint angular velocity significantly decreases (e.g., falls below a preset threshold, such as 0.5 rad/s), the system determines entry into the stance phase. This is because the stable acceleration and low knee joint angular velocity reflect static motion characteristics after the foot-ground contact, contrasting with dynamic changes in the swing phase.

The switching condition from the stance phase to the swing phase is as follows. When the sensors detect the foot lifting off the ground, for example when the thigh acceleration exhibits significant fluctuations (e.g., within ±10% range), and when the amplitude of the knee joint angular velocity increases (e.g., exceeds a preset threshold, such as 1.0 rad/s), the system determines entry into the swing phase. This is because the acceleration fluctuations and increased knee joint angular velocity indicate the foot leaves the ground and enters into a dynamic motion state.

The determination condition for the unknown gait phase is as follows. When the sensor data does not clearly meet characteristic conditions of the stance phase or the swing phase, for example when the combination of the acceleration measured at the thigh and the knee joint angular velocity falls within a fuzzy interval of the predefined threshold (e.g., the knee joint angular velocity fluctuates within 0.5-1.0 rad/s while the acceleration remains unstable), or when data noise interference causes ambiguous features, an unknown gait phase is determined. This serves as the algorithm's default handling for transitional or uncertain states, typically occurring at gait phase switching boundaries or during algorithm initialization.

These unadjusted switching conditions generally rely on fixed thresholds and direct trend analysis of sensor data, suiting common gait patterns (e.g., level-ground walking).

Furthermore, after the activity type changes, the gait phase switching conditions undergo adjustments. Specifically when specific activities (e.g., ascending, descending, squatting) are detected, the gait phase switching conditions are dynamically adjusted according to activity characteristics to enhance detection accuracy and adaptability. In some embodiments, specific adjustment methods are described below.

An activity recognition module extracts the activity features (e.g., knee joint angle, shank tilt angle, hip projection deviation) to characterize the current motion context.

The thresholds or feature weights are adjusted according to activity types. For example, a higher knee joint angular velocity threshold may be required during ascending, while a longer duration may be required for the stance phase during squatting.

In some embodiments, during ascending, the switching conditions are manifested as follows.

Regarding the switching condition from swing to stance, it requires a shorter stabilization time for the acceleration measured at the thigh (e.g., reduced from 200 ms to 150 ms) and a higher peak knee joint angular velocity (e.g., adjusted from 1.0 rad/s to 1.5 rad/s) to accommodate faster foot landing.

Regarding the switching condition from stance to swing, a larger knee joint angle change (e.g., exceeding 60°) is detected as an additional condition, reflecting leg lifting.

In some embodiments, during descending, the switching conditions are manifested as follows.

Regarding the switching condition from swing to stance, the knee joint angular velocity threshold is reduced (e.g., adjusted from 1.0 rad/s to 0.8 rad/s) to accommodate a slower lowering motion.

Regarding the switching condition from stance to swing, the acceleration fluctuation detection window is prolonged (e.g., from 200 ms to 300 ms) to capture a smooth transition.

In some embodiments, during squatting, the switching conditions are manifested as follows.

The stance phase is prolonged. This requires the duration of a stable acceleration and a significantly reduced knee joint angle (e.g., less than 120°) to exceed a threshold (e.g., 500 ms), thereby avoiding a falsely recognized brief pause.

Through dynamic adjustments of thresholds and time windows, the switching conditions can better match the motion characteristics of specific activities, thereby preventing false recognition. For example, during ascending, it is prevented from being falsely recognized as a swing phase due to fast landing, and during squatting, it is prevented from being falsely recognized as swing initiation due to prolonged stance.

Furthermore, to ensure seamless control for gait phase switching, the following control logic is adopted.

A smooth transition mechanism is applied. An overlapping time windows (e.g., 50-100 ms) is applied at a gait phase switching boundary for motion feature smoothing. For example, sliding averages (e.g., 5-point or 10-point moving averages) are applied to the thigh acceleration and the knee joint angular velocity to reduce switching jitter caused by noise or transient fluctuations.

When potential switching (e.g., from stance to swing) is detected, the algorithm temporarily maintains the current gait phase state (e.g., for an additional 50 ms) before confirming the new gait phase, thereby verifying persistence of feature trends.

State persistence check is performed. This requires the feature to satisfy switching conditions beyond a minimum duration (e.g., 100 ms), avoiding false triggers from transient anomalies (e.g., sensor jitter). For example, the algorithm confirms switching from the stance phase to the swing phase only when the knee joint angular velocity continuously exceeds the threshold and a continuous acceleration fluctuation occurs.

Dynamic threshold update is performed. The threshold is adjusted in real time according to the activity type to ensure the switching condition aligns with the current motion context. For example, the angular velocity threshold is increased during running to match a faster swing frequency. When the activity type changes (e.g., from walking to ascending), the algorithm updates the threshold within a first full gait cycle after detecting a new activity, thereby preventing false recognition of the gait phase during the transition.

A fallback mechanism is adopted. The algorithm can fall back to the previous gait phase within a short time (e.g., within 100 ms) and label it as an unknown gait phase if an anomaly is detected after switching (e.g., the acceleration is suddenly stabilized after switching to the swing phase). Redetermination is performed after the data is stabilized. This mechanism is particularly applicable to transitional states or unstable sensor signals.

Regarding the outcomes of seamless control, the above logic ensures gait phase switching continuity and accuracy, preventing control signal interruptions. For example, in exoskeleton or walking aid applications, smooth gait phase switching is directly translated to seamless motor control responses, enhancing wearer comfort and safety.

Furthermore, in an embodiment, the activity recognition process is as follows.

Feature comparison is performed based on a threshold rule. The motion features are compared based on the predefined threshold rule to determine whether the requirement for a specific activity is met.

Extreme value detection is performed within a time window. The motion feature extreme (e.g., maximum or minimum value) is analyzed through a time window (e.g., 100-350 ms) to capture a dynamic motion change. For example, it is determined whether there is a feature pattern related to ascending or descending by detecting a maximum shank tilt angle and a peak hip projection deviation. Then, temporal condition filtering (e.g., peak occurrence duration) is performed to ensure detection robustness.

Cumulative counting and state persistence check are performed. Cumulative counting is performed for motion features satisfying the preset condition, such as consecutive occurrences of peak shank angles exceeding a certain threshold or a duration when the knee joint angular velocity is maintained within a certain range. State persistence check (e.g., requiring a certain motion feature combination to continuously satisfy the condition beyond a set duration) can avoid false recognition caused by transient noise or abnormal data.

Dynamic update is performed. The motion features are dynamically updated during the stance phase, for example by calculating the product of the knee joint angular velocity and the hip projection deviation or by performing feature integration based on the time window to characterize the motion trend and intensity. These dynamically updated motion features can distinguish the activity types. For example, ascending may exhibit larger knee joint angle variations and negative hip projection deviations, while descending may demonstrate smoother knee joint and thigh angular velocities as well as specific shank angle ranges.

A conditional logic combination (e.g., "AND", "OR") is employed to jointly analyze multiple motion features. For example, if the knee joint angle exceeds a certain threshold while the knee joint angular velocity shows a positive trend and the hip projection deviation is below a negative value, the activity may be determined as ascending. The logic combination avoids complex computational models, relying solely on basic mathematical operations (e.g., addition, subtraction, multiplication, division, comparison) to achieve lightweight processing.

Therefore, in the present disclosure, the activity recognition process constitutes a lightweight analysis approach based on the data trends of the motion parameter sensors. It does not rely on resource-intensive techniques (e.g., machine learning or deep learning), but rather performs direct feature extraction and threshold logic determination based on real-time data of the motion parameter sensors. The predefined rule and time window-based analyses reduce computational complexity, ensuring efficient operation on embedded devices. All feature calculations utilize low-overhead mathematical operations (e.g., addition, multiplication, comparison) combined with cached data within limited time windows to avoid large-scale data storage and processing.

The above lightweight analysis approach efficiently distinguishes multiple activity types. For example: For example:
Level walking exhibits stable knee joint angular velocity fluctuations and knee joint angle variations.

Ascending demonstrates larger knee joint angle variations, negative Y-direction hip projection deviations, and specific shank tilt angles.

Descending presents smooth knee joint angular velocities and smaller shank tilt angles.

Running exhibits high-frequency fluctuations in the knee joint angular velocity and significant enhancement of thigh acceleration. Specifically, the knee joint angular velocity demonstrates rapid and periodic positive-negative alternations during running, reflecting rapid leg flexion in the swing phase (leg lifting) and brief extension in the stance phase (landing). Meanwhile, thigh acceleration peaks during the swing phase, showing larger positive variations particularly when the leg strides forward, while accompanied by brief deceleration shocks upon landing. Knee joint angle variations exhibit large amplitudes with steady rhythm, and shank tilt angles frequently switch with strides, collectively presenting a highly dynamic motion pattern.

Squatting demonstrates significantly reduced knee joint angles and longer stance phase durations.

Backward walking shows an X-direction hip projection deviation tending toward negative and a Y-direction hip projection deviation tending toward positive, with the cumulative value of the knee joint angular velocity displaying a positive trend.

An unknown activity is recognized when the feature pattern fails to meet any known activity conditions.

The technical principles of the present disclosure are described with reference to the specific embodiments. The description is merely intended to explain the principles of the present disclosure, rather than to limit the protection scope of the present disclosure in any way. Based on the explanation herein, those skilled in the art may derive other specific implementations of the present disclosure without creative effort, but these implementations should fall within the protection scope of the present disclosure.

## Claims

1. A knee joint exoskeleton motion recognition method, **characterized by** being based on a lower-limb exoskeleton device, wherein the lower-limb exoskeleton device comprises: a thigh extension frame (2) and a shank extension frame (3) strapped to a thigh and a shank, respectively; the thigh extension frame (2) and the shank extension frame (3) are relatively rotatable around a knee joint; the lower-limb exoskeleton device is provided with a motion parameter sensor; and the knee joint exoskeleton motion recognition method comprises a following process:
acquiring, by the motion parameter sensor, a motion parameter of the lower-limb exoskeleton device to determine a motion feature, and determining a motion state based on the motion feature;
wherein the motion feature comprises a knee joint angle, a knee joint angular velocity, a tilt angle of the shank and/or the thigh, an angular velocity of the shank and/or the thigh, an acceleration of the thigh and/or the shank, and a hip projection deviation; and the hip projection deviation is defined as a change in a hip position at a current state relative to a preset initial posture of a lower limb.

2. A knee joint assistance device, **characterized by** comprising: a lower-limb exoskeleton device, wherein the lower-limb exoskeleton device comprises:
a joint drive motor (1), wherein the joint drive motor (1) comprises a pair of rotating assemblies, and the joint drive motor (1) is configured to apply a torque to the pair of rotating assemblies configured to rotate relatively to each other; and a center of relative rotation of the rotating assemblies corresponds to a knee joint;
a thigh extension frame (2) and a shank extension frame (3) connected to the pair of rotating assemblies, respectively, wherein the thigh extension frame (2) and the shank extension frame (3) are strapped to a thigh and a shank, respectively; and
a motion parameter sensor disposed on the lower-limb exoskeleton device and configured to acquire a motion parameter of the lower-limb exoskeleton device to determine a motion feature, wherein the motion feature comprises a knee joint angle, a knee joint angular velocity, a tilt angle of the shank and/or the thigh, an angular velocity of the shank and/or the thigh, an acceleration of the thigh and/or the shank, and a hip projection deviation; and the hip projection deviation is defined as a change in a hip position at a current state relative to a preset initial posture of a lower limb.

3. The knee joint assistance device according to claim 2, **characterized in that** the motion parameter sensor comprises a pair of inertial measurement units (IMUs) disposed on the thigh extension frame (2) and the shank extension frame (3), respectively.

4. The knee joint assistance device according to claim 2, **characterized in that** the motion parameter sensor comprises an IMU disposed on the thigh extension frame (2) or the shank extension frame (3) and a knee joint angle sensor.

5. The knee joint assistance device according to claim 2, **characterized by** further comprising:
a thigh strapping device (21), wherein the thigh strapping device (21) is disposed on the thigh extension frame (2) and strapped to the thigh; and
a first shank strapping device (31) and a second shank strapping device (32), wherein the first shank strapping device (31) and the second shank strapping device (32) are disposed on the shank extension frame (3) and strapped between a calf and the knee joint.

6. The knee joint assistance device according to claim 5, **characterized in that** the thigh strapping device (21) comprises an upper connection seat (211) and a first strapping component (212); the upper connection seat (211) is rotatably disposed on the thigh extension frame (2); a rotation axis of the upper connection seat (211) is horizontally oriented; the upper connection seat (211) is attached to a posterior side of the thigh; and the first strapping component (212) straps the thigh to the upper connection seat (211).

7. The knee joint assistance device according to claim 5, **characterized in that** the first shank strapping device (31) comprises a lower connection seat (311) and a second strapping component (312); the lower connection seat (311) is rotatably disposed on the shank extension frame (3); a rotation axis of the lower connection seat (311) is horizontally oriented; the lower connection seat (311) is attached to a posterior side of the shank; and the second strapping component (312) straps the shank to the lower connection seat (311).

8. The knee joint assistance device according to claim 5, **characterized in that** the second shank strapping device (32) comprises a knee connection seat (321) and a third strapping component (322); the knee connection seat (321) is disposed on the shank extension frame (3); the knee connection seat (321) is attached to a lateral side of the shank; the third strapping component (322) straps the shank to the knee connection seat (321); and the knee connection seat (321) is located above a lower connection seat (311).

9. The knee joint assistance device according to claim 5, **characterized in that** the joint drive motor (1) is located at a lateral side of the knee joint; the thigh extension frame (2) and the shank extension frame (3) are each an integrally curved plate; an end of the thigh extension frame (2) and an end of the shank extension frame (3) away from the joint drive motor (1) extend to a posterior side of a leg; and the thigh strapping device (21) and the first shank strapping device (31) are disposed on the end of the thigh extension frame (2) and the end of the shank extension frame (3) away from the joint drive motor (1), respectively.

10. The knee joint assistance device according to claim 2, **characterized by** further comprising: a power module, wherein the power module is electrically connected to the joint drive motor (1), and the power module is located at or above a waist.
